# EUROPÄISCHE PATENTSCHRIFT

(11) **EP 1 512 416 B1**
(45) Veröffentlichungstag und Bekanntmachung des Hinweises auf die Patenterteilung: **03.09.2008**
(21) Anmeldenummer: 04026912.8
(22) Anmeldetag: 13.11.2002
(51) Int. Cl.: A61L 2/26, A61B 19/02, A61J 1/00

(54) **Sterilbehälter**
Sterilizing container
Contenant sterile

(30) Priorität: 15.11.2001 DE 10156937; 06.03.2002 DE 10210905
(43) Veröffentlichungstag der Anmeldung: 09.03.2005
(62) Teilanmeldung aus: 02799720.4
(73) Patentinhaber: AESCULAP AG & Co. KG, 78532 Tuttlingen (DE)
(72) Erfinder: Gleichauf, Wilhelm, 78532 Tuttlingen-Möhringen (DE); Jakab, Mariana, 78532 Tuttlingen (DE); Oertmann, Friedrich-Wilhelm, 78532 Tuttlingen (DE); Renner, Torsten, 07607 Eisenberg (DE); Schuster, Stefan, 78048 Villingen-Schwenningen (DE); Schwanke, Wolfgang, verstorben (DE)
(74) Vertreter: Hoeger, Stellrecht & Partner Patentanwälte

(56) Entgegenhaltungen:
- WO-A-01/08583
- DE-A- 3 407 112
- US-A- 4 728 504
- US-A- 5 176 884
- US-A- 5 183 643
- US-A- 5 324 489

## Beschreibung

Die Erfindung betrifft einen Sterilbehälter, insbesondere zur Aufnahme und sterilen Aufbewahrung von chirurgischem Besteck oder Material, umfassend einen durch einen Behälterboden und Behälterwände gebildeten Aufnahmeraum, einen Deckel zum Verschließen des Aufnahmeraums und eine Gasaustauschöffnung, welche von einem in einem Filterhalter gehaltenen Sterilfilter verschließbar ist, wobei das Sterilfilter und der Filterhalter eine Filtereinheit bilden und wobei der Filterhalter am Deckel gelagert ist, wobei das Sterilfilter an einem Trägerelement gehalten ist, wobei das Trägerelement ein erstes und ein zweites Stützelement umfasst und wobei das Sterilfilter zwischen den beiden Stützelementen gehalten ist.

Derartige Sterilbehälter werden zusammen mit beispielsweise darin gelagertem chirurgischem Besteck oder Material in einem Sterilisator sterilisiert. Anschließend können sie in einen sterilen Operationsbereich gebracht und dort geöffnet werden. Das Sterilfilter ist erforderlich, um nach dem Sterilisationsvorgang das Eindringen von Keimen in den Aufnahmeraum des Behälters zu verhindern. Dieses Filter muß jedoch von Zeit zu Zeit ausgetauscht werden. Dies ist bei bekannten Sterilbehältern nur umständlich möglich. Außerdem ist die Herstellung eines Deckels mit einstückig daran angeordnetem Filterhalter sehr aufwendig.

Beispiele für Sterilbehälter, insbesondere zur Aufnahme und sterilen Aufbewahrung von chirurgischem Besteck oder Material, sind aus der US 5,176,884, der DE 3 407 112 A1, der US 5,324,489 sowie der US 5,183,643 bekannt.

Daher ist es Aufgabe der vorliegenden Erfindung, einen Sterilbehälter der eingangs beschriebenen Art so zu verbessern, daß eine Stabilität der Filtereinheit erhöht wird.

Diese Aufgabe wird bei einem Sterilbehälter der eingangs beschriebenen Art erfindungsgemäß dadurch gelöst, daß das Trägerelement ein erstes und ein zweites stützelement (42,43) umfaßt und wobei das sterilfilter (52) zwischen den beiden Stützelementen (42,43) gehalten ist, wobei mindestens eines der beiden Stützelemente die Gasaustauschöffnung mindestens teilweise übergreifende Auflageabschnitte zum Stützen des Sterilfilters umfaßt.

Diese Ausgestaltung ermöglicht es, gegebenenfalls die Filtereinheit komplett auszutauschen. Dazu muß insbesondere beim Einlegen das Sterilfilter nicht mehr selbst gegriffen werden, wodurch einerseits eine Beschädigung desselben vermieden, andererseits eine Beschmutzung verhindert wird. Die Herstellung des Deckels wird zusätzlich vereinfacht, da der Filterhalter nachträglich am Deckel gelagert werden kann. Um Beschädigungen des Sterilfilters zu vermeiden, wird das Sterilfilter an einem Trägerelement gehalten. Zur Erhöhung der Stabilität und zur Erhaltung der Form des Sterilfilters umfaßt das Trägerelement ein erstes und ein zweites Stützelement und das Sterilfilter zwischen den beiden Stützelementen gehalten ist. Das Sterilfilter läßt sich dadurch sicher am Trägerelement ergreifen, ohne daß das Filter beschmutzt oder beschädigt wird. Zur weiteren Erhöhung der Stabilität der Filtereinheit ist vorgesehen, daß mindestens eines der beiden Stützelemente die Gasaustauschöffnung mindestens teilweise übergreifende Auflageabschnitte zum Stützen des Sterilfilters umfaßt. Dadurch wird zwar die Gasaustauschöffnung verkleinert, freie Flächen des Sterilfilters jedoch auch, so daß sich dessen Form stabilisieren läßt, was insbesondere bei Beaufschlagung mit unter Druck stehendem Heißdampf vorteilhaft ist.

Vorteilhaft ist es, wenn das Sterilfilter und der Filterhalter lösbar verbindbar sind, wenn das Sterilfilter in einer Entnahmestellung vom Filterhalter lösbar und in einer Verbindungsstellung am Filterhalter gehalten ist. Auf diese Weise muß beim Filtertausch nur das Sterilfilter selbst erneuert werden und nicht die gesamte Filtereinheit. Zwar könnte die Filtereinheit insgesamt ausgetauscht werden, allerdings würde dann bei einem noch intakten Filterhalter dieser ersetzt, obwohl dies nicht erforderlich wäre. Dadurch wird insgesamt die Wirtschaftlichkeit des Sterilbehälters verbessert.

Gemäß einer bevorzugten Ausführungsform der Erfindung kann vorgesehen sein, daß das Sterilfilter, das erste und das zweite Stützelement unlösbar miteinander verbunden sind, insbesondere verklebt, verklemmt oder verschweißt. Somit wird eine insgesamt formstabile Einheit umfassend das Trägerelement und das Sterilfilter gebildet, wodurch der Austausch desselben erleichtert wird.

Das Trägerelement und der Filterhalter können auf verschiedene Weise miteinander verbunden sein, beispielsweise verschraubt oder verklemmt. Besonders vorteilhaft ist es jedoch, wenn eine Bajonettverbindung zum Verbinden des Trägerelements und des Filterhalters und zum Überführen des Trägerelements von der Entnahmestellung in die Verbindungsstellung vorgesehen ist. Das Trägerelement kann mit dem Sterilfilter einfach verbunden werden, nämlich indem das Trägerelement zunächst in den Filterhalter eingesetzt und anschließend über einen bestimmten Winkelbereich verdreht wird, welcher im wesentlichen von der Anzahl der miteinander in Eingriff bringbaren Bajonettvorsprünge und -aufnahmen und deren Winkelerstreckung abhängt. Die Verbindungsstellung wird üblicherweise definiert durch relative Anschläge zwischen dem Trägerelement und dem Filterhalter, die eine Drehbewegung begrenzen.

Um eine definierte Verbindungsstellung zu gewährleisten und ein Lösen des Sterilfilters vom Filterhalter zu verhindern, ist es günstig, wenn ein Verriegelungsmechanismus vorgesehen ist zum Verriegeln der Verbindungsstellung des Sterilfilters und des Filterhalters.

Besonders einfach läßt sich ein Verriegelungsmechanismus durch eine Rastverbindung realisieren.

Bei einer weiteren vorteilhaften Ausführungsform der Erfindung kann vorgesehen sein, daß die Filtereinheit eine Abdeckung umfaßt zum einseitigen Überdecken des Sterilfilters. Dadurch wird das Sterilfilter einseitig geschützt gegen Beschädigungen, beispielsweise durch die im Sterilbehälter gelagerten Gegenstände.

Um einen optimalen Gasaustausch zu gewährleisten und gleichzeitig das Sterilfilter sicher gegen Beschädigungen zu schützen, kann es von Vorteil sein, wenn die Abdeckung mit Durchbrechungen versehen ist zum Ermöglichen eines Gasaustausches durch die Durchbrechungen hindurch und wenn die Durchbrechungen in einer Richtung quer zu einer Durchlaßrichtung mit Durchbrechungsabdeckungen bedeckt sind.

Vorzugsweise ist die Abdeckung vom Sterilfilter beabstandet. Dadurch wird verhindert, daß das Sterilfilter bei einer Beaufschlagung mit Heißdampf nicht in Kontakt mit der Abdeckung kommt, falls sich das Sterilfilter verformt, beispielsweise wölbt.

Die Abdeckung und der Filterhalter könnten zwar einstückig ausgebildet sein, bei einer bevorzugten Ausführungsform der Erfindung kann aber vorgesehen sein, daß die Abdeckung mit dem Filterhalter lösbar verbindbar ist, daß die Abdeckung in einer Abnehmstellung vom Filterhalter abnehmbar ist und in einer Verschlußstellung am Filterhalter gehalten ist. Die Abdeckung läßt sich so auf einfache Weise entfernen, um das Sterilfilter auszutauschen.

Die Abdeckung könnte beispielsweise mit Schrauben oder Klemmen am Filterhalter befestigt werden, allerdings ist es besonders vorteilhaft, wenn eine zweite Bajonettverbindung zum Verbinden der Abdeckung und des Filterhalters und zum Überführen der Abdeckung von der Abnehmstellung in die Verschlußstellung vorgesehen ist. Eine Verbindung könnte beispielsweise hergestellt werden, indem die Abdeckung mit entsprechenden Vorsprüngen in Aufnahmen des Filterhalters eingeführt und anschließend als Ganzes relativ zum Filterhalter verdreht wird. Umgekehrt läßt sich die Abdeckung auf einfache Weise entfernen.

Zum Vermeiden eines unbeabsichtigten Lösens der Abdeckung vom Filterhalter ist ein zweiter Verriegelungsmechanismus vorgesehen zum Verriegeln der Abdeckung und des Filterhalters in der Verschlußstellung.

Der zweite Verriegelungsmechanismus könnte beispielsweise mittels eines separat betätigbaren Verriegelungselements realisiert werden. Besonders einfach ist es jedoch, wenn der zweite Verriegelungsmechanismus eine zweite Rastverbindung umfaßt. Dabei sind alle Arten von Rastverbindungen möglich, beispielsweise federnd gelagerte Rastvorsprünge, die in korrespondierende Ausnehmungen eingreifen.

Ferner ist es von Vorteil, wenn das Trägerelement ein mit der Abdeckung in Eingriff bringbares Zentrierelement umfaßt zum Zentrieren der Abdeckung am Filterhalter. Damit wird insbesondere das Aufsetzen der Abdeckung erleichtert und vereinfacht. Außerdem kann die Zentrierhilfe auch als Anschlag dienen, um eine Beschädigung des Sterilfilters durch die Abdeckung vermeiden zu helfen.

Um einen Gasaustausch zwischen dem Aufnahmeraum und der Umgebung des Sterilbehälters zusätzlich zu steuern und/oder zu regeln, kann es günstig sein, wenn die Filtereinheit bewegbar gelagert ist, wenn die Filtereinheit in einer Schließstellung einen Strömungspfad schließt und in einer Durchlaßstellung den Strömungspfad öffnet, so daß ein Gasaustausch in der Schließstellung nur durch das Sterilfilter und in der Durchlaßstellung durch das Sterilfilter und/oder durch den Strömungspfad ermöglicht wird. Damit kann insbesondere dann, wenn ein größerer Gasaustausch erforderlich ist, die Filtereinheit in die Durchlaßstellung überführt und ein zusätzlicher Strömungspfad geöffnet werden.

Besonders einfach wird der Zusammenbau des Sterilbehälters, wenn Lagerungselemente am Filterhalter und am Deckel zum Lagern der Filtereinheit am Deckel vorgesehen sind.

Ein besonders einfacher Aufbau des Sterilbehälters mit einer beweglich gelagerten Filtereinheit ergibt sich in vorteilhafter Weise, wenn die Lagerungselemente mindestens einen Lagerbolzen und eine zugeordnete Lagerbuchse umfassen, wenn der Lagerbolzen jeweils endseitig einen Anschlag aufweist zum Begrenzen einer Bewegung der Lagerbuchse relativ zum Lagerbolzen und wenn der Lagerbolzen am Deckel und die Lagerbuchse am Filterhalter oder umgekehrt angeordnet sind.

Keine zusätzlichen Teile sind erforderlich, wenn einer der beiden Anschläge durch den Deckel und der andere Anschlag durch einen Kopf des Lagerbolzens gebildet wird.

Einen zusätzlichen Schutz der Lagerungselemente erhält man, wenn die Abdeckung die Lagerungselemente überdeckt. Auf diese Weise sind die Lagerungselemente vor einer unbeabsichtigten Manipulation geschützt.

Um einen zusätzlichen Strömungspfad zu schließen und dadurch eine keimfreie Aufbewahrung im Sterilbehälter gelagerter Gegenstände zu ermöglichen, ist die Filtereinheit in der Schließstellung am Sterilbehälter unter Vorspannung gehalten.

Eine solche Vorspannung läßt sich besonders einfach realisieren, wenn sich ein in Längsrichtung des Lagerbolzens wirkendes vorspannendes Element zwischen einem der beiden Anschläge und der Lagerbuchse abstützt. Als vorspannendes Element können beliebige elastische Elemente, zum Beispiel Spiralfedern oder gummielastische Elemente, verwendet werden.

Vorteilhaft ist es, wenn ein Dichtelement zur gasdichten Lagerung der Filtereinheit am Deckel vorgesehen ist. Dadurch läßt sich eine vollständige Abdichtung der Filtereinheit am Deckel realisieren, so daß ein Gasaustausch nur durch das Sterilfilter hindurch möglich ist.

Besonders günstig ist es, wenn das Dichtelement einen am Trägerelement gelagerten Dichtring umfaßt. Dichtringe sind besonders einfach herzustellen und auf einfache Weise am Trägerelement anzuordnen.

Gemäß einer weiteren bevorzugten Ausführungsform der Erfindung ist es von Vorteil, wenn ein Überdruckventil vorgesehen ist, wenn das Überdruckventil so angeordnet ist, daß es in einer Grundstellung eine Schließstellung einnimmt und wenn es eine Durchlaßstellung einnimmt, wenn ein Druck in einer Umgebung des Sterilbehälters einen Druck im Sterilbehälter um eine vorgegebene Druckdifferenz übersteigt. Auf diese Weise läßt sich eine Beschädigung des Sterilbehälters vermeiden, denn bevor der Sterilbehälter aufgrund eines besonders großen Drucks in der Umgebung oder eines entsprechend hohen Druckgradienten verformt werden kann, öffnet das Überdruckventil und ermöglicht das Einströmen von Gas in den Behälter.

Keine zusätzlichen Bauelemente und weitere Durchbrechungen sind erforderlich, wenn die Filtereinheit das Überdruckventil bildet. Die Filtereinheit übernimmt damit zwei Funktionen, einerseits dient sie dem Zurückhalten von Keimen, andererseits als Überdruckventil.

Vorzugsweise ist ein die Filtereinheit beabstandet überdeckendes Schutzelement vorgesehen. Damit läßt sich insbesondere das Sterilfilter gegen eine mechanische Beschädigung sicher schützen.

Besonders günstig ist es, wenn die Filtereinheit auf einer Innenseite des Deckels gelagert ist und wenn das Schutzelement auf einer Außenseite des Deckels angeordnet ist. Damit ist die Filtereinheit mit dem Sterilfilter von beiden Seiten gegen mechanische Beanspruchungen sicher geschützt.

Um eine Beaufschlagung des Sterilfilters mit Flüssigkeit zu minimieren, ist es vorteilhaft, wenn zwischen dem Schutzelement und dem Deckel mindestens eine mit der Gasaustauschöffnung in Fluidverbindung stehende Gasdurchtrittsöffnung vorgesehen ist, die so angeordnet ist, daß eine Gasströmung in einer im wesentlichen quer zur Durchlaßrichtung des Sterilfilters verlaufenden Strömungsrichtung ermöglicht wird. Auf diese Weise kann Gas, beispielsweise Heißdampf zur Sterilisation, auf einfache Weise durch das Sterilfilter hindurchgelangen, wohingegen Flüssigkeiten zunächst vom Sterilfilter ferngehalten werden.

Um es Flüssigkeiten zusätzlich zu erschweren, in die Filtereinheit einzudringen, ist es günstig, wenn eine auf der Außenseite des Deckels angeordnete und von der Gasaustauschöffnung weg weisende, nach außen relativ zu einer Deckelebene abfallende Einströmkante vorgesehen ist. Gelangt Flüssigkeit auf den Deckel, so kann sie aufgrund der Neigung der Einströmkante von der Gasaustauschöffnung weg fließen und somit nicht auf die Filtereinheit gelangen.

Um den Einbau des Filterhalters zu erleichtern und die Stabilität desselben zu erhöhen, ist bei einer bevorzugten Ausführungsform der Erfindung vorgesehen, daß der Filterhalter verdrehgesichert am Deckel gelagert ist.

Vorzugsweise ist das Sterilfilter ein Dauerfilter, insbesondere ein aus Polytetrafluorethylen (PTFE) hergestelltes. Damit lassen sich Wartungsintervalle des Sterilbehälters verlängern, was einen Austausch der Filtereinheit nur noch selten erforderlich macht.

Besonders günstig und einfach in der Herstellung wird der Sterilbehälter dann, wenn der Deckel aus einem Kunststoff hergestellt ist, insbesondere aus Polyetheretherketon (PEEK) oder Polyphenylensulfon (PPSU).

Die nachfolgende Beschreibung einer bevorzugten Ausführungsform der Erfindung dient im Zusammenhang mit der Zeichnung der näheren Erläuterung. Es zeigen:
- Figur 1:: Eine Explosionsdarstellung einer zerlegten, dreiteiligen Filtereinheit;
- Figur 2:: eine Schnittansicht längs Linie 2-2 in Figur 1 und
- Figur 3:: eine Schnittansicht längs Linie 3-3 in Figur 1.

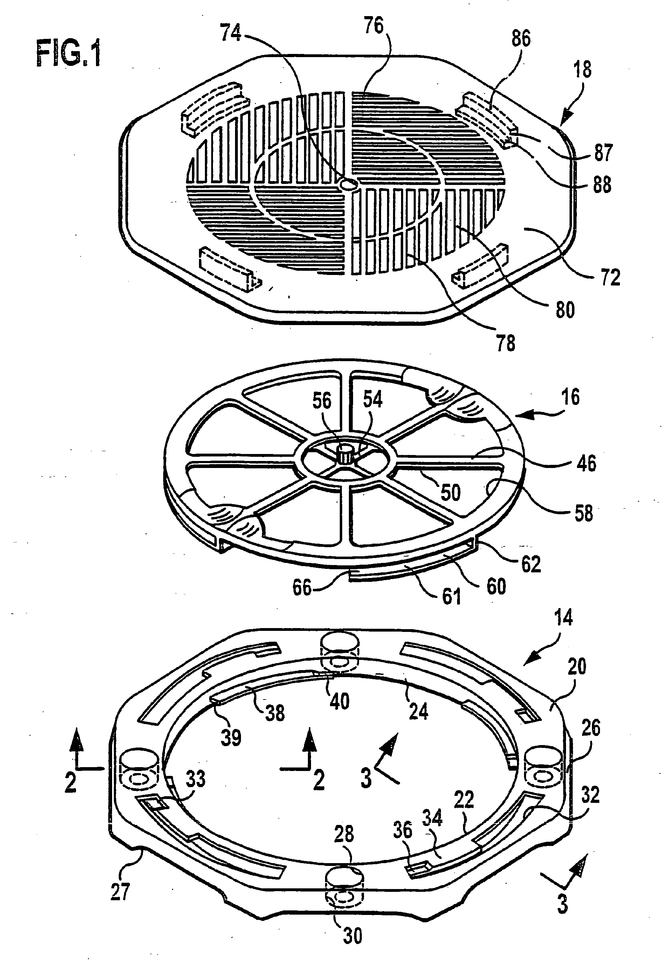

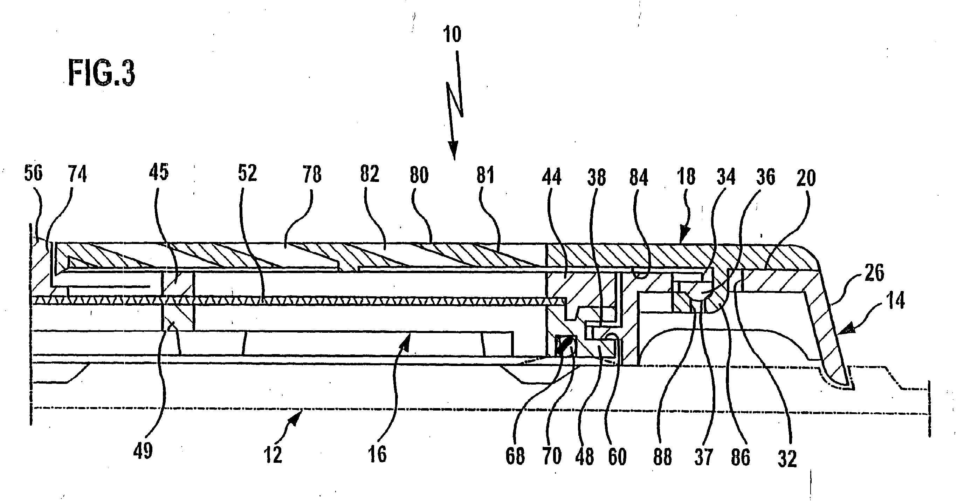

In den Figuren 1 bis 3 ist eine insgesamt mit dem Bezugszeichen 10 versehene, dreiteilige Filtereinheit dargestellt, die an einem Deckel 12 eines nicht näher dargestellten Sterilbehälters gelagert ist.

Die Filtereinheit 10 umfaßt einen mit dem Deckel 12 verbindbaren Halterahmen 14, ein in den Halterahmen 14 einlegbares und mit diesem verbindbares Filterelement 16 sowie eine mit dem Halterahmen 14 verbindbare Abdeckung 18.

Der Halterahmen 14 umfaßt eine eine achteckige Außenkontur aufweisende Deckplatte 20, welche eine kreisförmige Öffnung 22 aufweist, die von einem umlaufenden Innenrand 24 umgeben ist. Ein umlaufender Außenrand 26 schließt sich an die achteckige Außenkontur der Deckplatte 20 an, so daß ein im Querschnitt im wesentlichen U-förmiges Hohlprofil gebildet wird. In Umfangsrichtung um jeweils 90° versetzt sind in die Deckplatte vier identische, als Lagerbuchsen 28 dienende zylindrische Hülsen eingesetzt, die an ihrem von der Deckplatte 20 entfernten Ende einen parallel zur Deckplatte 20 verlaufenden, nach innen weisenden Ringflansch 30 aufweisen. Zwischen den Lagerbuchsen 28 sind vier identische, sich etwa über einen Winkelbereich von 45° erstreckende Schlitze 32 angeordnet, die teilweise von einem einstückig mit der Deckplatte 20 gebildeten Vorsprung 34 überdeckt werden, an den sich eine in Umfangsrichtung weisende, angeformte federnde Rastnase 36 anschließt, die senkrecht zur Deckplatte 20 ein wenig bewegbar ist und auf einer von der Deckplatte 20 weg weisenden Seite einen nicht dargestellten kleinen kugelförmigen Vorsprung aufweist. Der Vorsprung 34 und die Rastnase 36 überdecken etwa 40 % des Schlitzes 32 in Umfangsrichtung von einem Ende des Schlitzes 32 ausgehend.

Ferner ist der Innenrand 24 mit vier symmetrisch über den Umfang verteilten, sich in Umfangsrichtung erstreckenden und radial nach innen vorstehenden Radialvorsprüngen 38 versehen, die sich in etwa über einen Winkelbereich von 40° erstrecken. Einseitig sind die Radialvorsprünge 38 mit parallel versetzt abstehenden Rastzungen 40 verlängert, welche senkrecht zur Deckplatte 20 ein wenig bewegbar sind.

Eine freie, von der Deckplatte 20 weg weisende Randlinie 27 des Außenrandes 26 ist teilweise gewölbt und korrespondierend zu einer nicht näher dargestellten inneren Oberfläche des Deckels 12 ausgebildet, wodurch sich eine formschlüssige Anlage des Halterahmens 14 an den Deckel 12 realisieren läßt.

Das Filterelement 16 umfaßt zwei Klemmscheiben 42 und 43, die einen jeweils über acht sich radial erstreckende Stege 46 beziehungsweise 50 mit einem Außenring 44 beziehungsweise 48 verbundenen Innenring 45 beziehungsweise 49 aufweisen. Der Innenring 45 der Klemmscheibe 42 wird von vier sich kreuzförmig angeordneten Haltestegen 54 durchsetzt, die zentral einen Zentriervorsprung 56 tragen. Zwischen den Klemmscheiben 42 und 43 ist ein Sterilfilter 52 eingelegt, das die von den Außenringen 44 und 48 begrenzte ringförmige Öffnung 58 vollständig überdeckt. Die beiden Klemmscheiben 42 und 43 sowie das Sterilfilter 52 sind fest miteinander verbunden, beispielsweise verklebt oder ultraschallverschweißt, und bilden eine einzige Einheit.

Der Außendurchmesser des ringförmigen Filterelements 16 entspricht dem Innendurchmesser der Öffnung 22 des Halterahmens 14 und kann daher in diese Öffnung 22 eingesetzt werden. Zur Verbindung des Filterelements 16 mit dem Halterahmen 14 ist der Außenring 48 mit vier symmetrisch über den Umfang verteilten Aufnahmenuten 60 versehen, die radial nach außen geöffnet sind. Jeweils ein seitliches Ende der Aufnahmenut 60 ist mit einer Nutendwand 62 verschlossen. Das Filterelement 16 wird in den Halterahmen 14 derart eingesetzt, daß die vier Aufnahmenuten 60 zwischen die mit den Radialvorsprüngen 38 versehenen Bereiche des Innenrandes 24 eingreifen. Ein radial abstehender Außenringflansch 64 des Außenrings 48 liegt in dieser Stellung auf den Radialvorsprüngen 38 auf. Das Filterelement 16 wird nun soweit verdreht, bis die Nutendwand 62 an die Stirnkante 39 des in die Aufnahmenut 60 eingreifenden Radialvorsprungs 38 anschlägt. In dieser Verbindungsstellung des Halterahmens 14 und des Filterelements 16 tauchen die an der Rastzunge 40 angeordneten Vorsprünge in korrespondierende Ausnehmungen 66 am offenen Ende jeweils einer Seitenwand 61 der Aufnahmenut ein, wodurch sich eine rastende Verriegelung der Verbindungsstellung ergibt.

Ferner ist der Außenring 48 mit einer vom Außenring 44 weg weisend geöffneten Ringnut 68 versehen, in die ein Dichtring 70 eingelegt ist.

Die Filtereinheit 10 wird mittels der Abdeckung 18 verschlossen, die eine zur Außenkontur der Deckplatte 20 korrespondierende achteckige Grundplatte 72 aufweist, die mit einer Zentralbohrung 74 zum Einführen des Zentriervorsprungs 56 versehen ist. Ein kreisförmiger Flächenbereich 76 ist mit in Viertelkreissektoren jeweils parallel verlaufenden Schlitzen 78 unterschiedlicher Länge versehen, so daß Stege 80 zwischen den Schlitzen 78 verbleiben, die relativ zur Grundplatte 72 schräg, etwa in einem Winkel von 60° verlaufend verlängert abstehen. Schräg verlaufende Stegbereiche 81 überdecken die Schlitze 78 jeweils vollständig, so daß jeweils schräg verlaufende Kanäle 82 gebildet werden. Ein direkter Durchgang senkrecht zur Grundplatte 72 durch die Schlitze 78 ist nicht möglich.

Von einer Unterseite 84 der Grundplatte 72 ragen vier symmetrisch verteilte, sich über einen Winkelbereich von etwa 30° erstreckende, im Querschnitt L-förmige Rückhaltevorsprünge 86 ab, deren einer freier Schenkel an seinem einen Ende eine senkrecht zur Grundplatte 72 verlaufende Bohrung 88 aufweist.

Die Abdeckung 18 wird mit dem Halterahmen 14 folgendermaßen verbunden: Die Abdeckung 18 wird mit den Rückhaltevorsprüngen 86 in nicht von Vorsprüngen 34 überdeckte Bereiche der Schlitze 32 eingeführt, bis die Unterseite 84 der Grundplatte 72 an der Deckplatte 20 anliegt. Durch Verdrehung der Abdeckung 18 im Uhrzeigersinn hintergreifen die freien Schenkel der Rückhaltevorsprünge 86 die Vorsprünge 34, bis eine Stirnseite 87 des Rückhaltevorsprungs 86 an eine Stirnkante 33 des Schlitzes 32 anschlägt. In dieser Verschlußstellung taucht eine an der Rastnase 36 angeordnete Halbkugel 37 in die Bohrung 88 ein und verriegelt die Verschlußstellung der Abdeckung 18 und des Halterahmens 14.

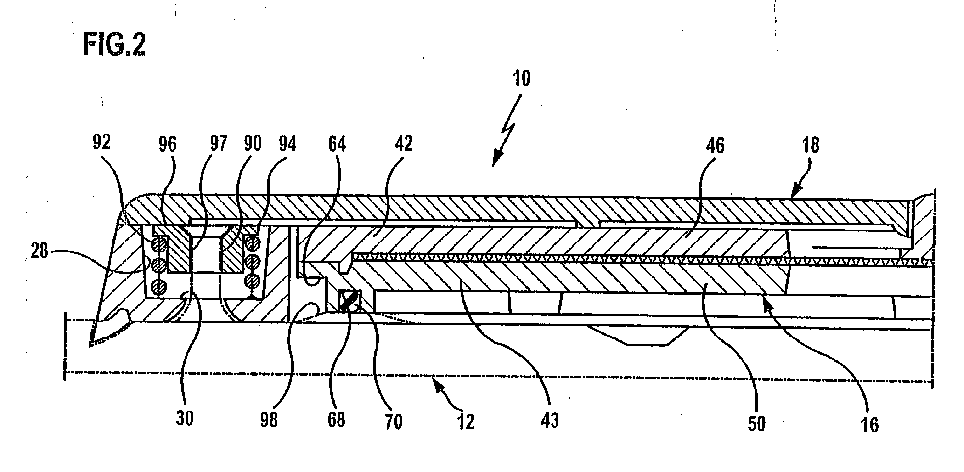

Im Zusammenhang mit Figur 2 wird die Befestigung des Halterahmens 14 am Deckel 12 erläutert. Am Deckel 12 sind benachbart einer nicht dargestellten kreisförmigen Durchbrechung auf seiner Innenseite vier im wesentlichen zylindrische Haltebolzen 90 senkrecht vom Deckel 12 weg abstehend angeordnet. Der Halterahmen 14 wird mit den Lagerbuchsen 28 über die Haltebolzen 90 geschoben, so daß die Ringflansche 30 die Haltebolzen 90 umgebend an der Innenseite des Deckels 12 anliegen. Jeder Haltebolzen 90 wird von einer Schraubenfeder 92 umgeben, die sich auf einer Seite am Ringflansch 30, auf der anderen Seite an einem Ringflansch 94 eines Deckels 96 abstützt, welcher eine zentrale Bohrung 97 aufweist, deren Innendurchmesser mit dem Außendurchmesser des Haltebolzen 90 korrespondiert. Der Deckel 96 ist mit dem Haltebolzen ultraschallverschweißt oder verklebt.

Aufgrund dieser Lagerung ist es möglich, die gesamte Filtereinheit 10 von der Innenseite des Deckels 12 gegen die Vorspannung der Schraubenfedern 92 abzuheben. Dies passiert beispielsweise dann, wenn ein Sterilbehälter mit einer daran angeordneten Filtereinheit 10 in einen Sterilisator eingebracht und mit Heißdampf unter Druck beaufschlagt wird. Der den Sterilbehälter umgebende Druck ist in diesem Fall höher als der Druck im Inneren des Behälters, so daß sich die gesamte Filtereinheit 10 vom Deckel 12 abhebt und einen zusätzlichen Strömungspfad öffnet zum Einströmen von Heißdampf. Dadurch wird verhindert, daß der Sterilbehälter durch zu große einwirkende Druckunterschiede verformt wird. Geht der von außen einwirkende Druck wieder zurück, wird die Filtereinheit 10 mittels der Schraubenfedern 92 gegen die Innenseite des Deckels 12 vorgespannt. Der Dichtring 70 liegt in dieser Schließstellung an einem entsprechenden Dichtvorsprung 98 des Deckels 12 an, so daß nur noch ein Gasaustausch durch das Sterilfilter 52 hindurch ins Innere des Sterilbehälters hinein möglich ist. Aufgrund dieser federnden Lagerung übernimmt die gesamte Filtereinheit 10 gleichzeitig auch die Funktion eines Überdruckventils.

## Patentansprüche

1. Sterilbehälter, insbesondere zur Aufnahme und sterilen Aufbewahrung von chirurgischem Besteck oder Material, umfassend einen durch einen Behälterboden und Behälterwände gebildeten Aufnahmeraum, einen Deckel (12) zum Verschließen des Aufnahmeraums und eine Gasaustauschöffnung, welche von einem in einem Filterhalter gehaltenen Sterilfilter verschließbar ist, wobei das Sterilfilter (52) und der Filterhalter (14) eine Filtereinheit (10) bilden und wobei der Filterhalter (14) am Deckel (12) gelagert ist, wobei das Sterilfilter (52) an einem Trägerelement (42, 43) gehalten ist, **dadurch gekennzeichnet, daß** das Trägerelement ein erstes und ein zweites Stützelement (42, 43) umfaßt und wobei das Sterilfilter (52) zwischen den beiden Stützelementen (42, 43) gehalten ist, wobei mindestens eines der beiden Stützelemente (42, 43) die Gasaustauschöffnung mindestens teilweise übergreifende Auflageabschnitte (46, 50) zum Stützen des Sterilfilters (52) umfaßt.

2. Sterilbehälter nach Anspruch 1, **dadurch gekennzeichnet, daß** das Sterilfilter (52) und der Filterhalter (14) lösbar verbindbar sind, daß das Sterilfilter (52) in einer Entnahmestellung vom Filterhalter (14) lösbar und in einer Verbindungsstellung am Filterhalter (14) gehalten ist.

3. Sterilbehälter nach einem der voranstehenden Ansprüche, **dadurch gekennzeichnet, daß** das Sterilfilter (52), das erste und das zweite Stützelement (42, 43) unlösbar miteinander verbunden sind, insbesondere verklebt, verklemmt oder verschweißt.

4. Sterilbehälter nach einem der Ansprüche 2 oder 3, **dadurch gekennzeichnet, daß** eine Bajonettverbindung (38, 60) zum Verbinden des Trägerelements (42, 43) und des Filterhalters (14) und zum Überführen des Trägerelements (42, 43) von der Entnahmestellung in die Verbindungsstellung vorgesehen ist.

5. Sterilbehälter nach einem der Ansprüche 2 bis 4, **dadurch gekennzeichnet, daß** ein Verriegelungsmechanismus (40, 66) vorgesehen ist zum Verriegeln der Verbindungsstellung des Sterilfilters (52) und des Filterhalters (14).

6. Sterilbehälter nach Anspruch 5, **dadurch gekennzeichnet, daß** der Verriegelungsmechanismus eine Rastverbindung (40, 66) umfaßt.

7. Sterilbehälter nach einem der voranstehenden Ansprüche, **dadurch gekennzeichnet, daß** die Filtereinheit (10) eine Abdeckung (18) umfaßt zum einseitigen Überdecken des Sterilfilters (52).

8. Sterilbehälter nach **dadurch gekennzeichnet, daß** die Abdeckung (18) mit Durchbrechungen (78) versehen ist zum Ermöglichen eines Gasaustausches durch die Durchbrechungen (78) hindurch und daß die Durchbrechungen (78) in einer Richtung quer zu einer Durchlaßrichtung mit Durchbrechungsabdeckungen (81) bedeckt sind.

9. Sterilbehälter nach einem der Ansprüche 7 oder 8, **dadurch gekennzeichnet, daß** die Abdeckung (18) vom Sterilfilter (52) beabstandet ist.

10. Sterilbehälter nach einem der Ansprüche 7 bis 9, **dadurch gekennzeichnet, daß** die Abdeckung (18) mit dem Filterhalter (14) lösbar verbindbar ist und daß die Abdeckung (18) in einer Abnehmstellung vom Filterhalter (14) abnehmbar und einer Verschlußstellung am Filterhalter (14) gehalten ist.

11. Sterilbehälter nach Anspruch 10, **dadurch gekennzeichnet, daß** eine zweite Bajonettverbindung (32, 34, 86) zum Verbinden der Abdeckung (18) und des Filterhalters (14) und zum Überführen der Abdeckung (18) von der Abnehmstellung in die Verschlußstellung vorgesehen ist.

12. Sterilbehälter nach einem der Ansprüche 10 oder 11, **dadurch gekennzeichnet, daß** ein zweiter Verriegelungsmechanismus (36, 37, 88) vorgesehen ist zum Verriegeln der Abdeckung (18) und des Filterhalters (14) in der Verschlußstellung.

13. Sterilbehälter nach Anspruch 12, **dadurch gekennzeichnet, daß** der zweite Verriegelungsmechanismus eine zweite Rastverbindung (36, 37, 88) umfaßt.

14. Sterilbehälter nach einem der Ansprüche 7 bis 13, **dadurch gekennzeichnet, daß** das Trägerelement (14) ein mit der Abdeckung (18) in Eingriff bringbares Zentrierelement (56) umfaßt zum Zentrieren der Abdeckung (18) am Filterhalter (14).

15. Sterilbehälter nach einem der voranstehenden Ansprüche, **dadurch gekennzeichnet, daß** die Filtereinheit (10) bewegbar gelagert ist, daß die Filtereinheit (10) in einer Schließstellung einen Strömungspfad schließt und in einer Durchlaßstellung den Strömungspfad öffnet, so daß ein Gasaustausch in der Schließstellung nur durch das Sterilfilter (52) und in der Durchlaßstellung durch das Sterilfilter (52) und/oder durch den Strömungspfad ermöglicht wird.

16. Sterilbehälter nach einem der voranstehenden Ansprüche, **dadurch gekennzeichnet, daß** Lagerungselemente am Filterhalter (14) und am Deckel (12) zum Lagern der Filtereinheit (10) am Deckel (12) vorgesehen sind.

17. Sterilbehälter nach Anspruch 16, **dadurch gekennzeichnet, daß** die Lagerungselemente mindestens einen Lagerbolzen (90) und eine zugeordnete Lagerbuchse (28) umfassen, daß der Lagerbolzen (90) jeweils endseitig einen Anschlag (94) aufweist zum Begrenzen einer Bewegung der Lagerbuchse (28) relativ zum Lagerbolzen (90) und daß der Lagerbolzen (90) am Deckel und die Lagerbuchse (28) am Filterhalter (14) oder umgekehrt angeordnet sind.

18. Sterilbehälter nach Anspruch 17, **dadurch gekennzeichnet, daß** einer der beiden Anschläge durch den Deckel (12) und der andere Anschlag durch einen Kopf (94) des Lagerbolzens (90) gebildet wird.

19. Sterilbehälter nach einem der Ansprüche 16 bis 18, **dadurch gekennzeichnet, daß** die Abdeckung (18) die Lagerungselemente überdeckt.

20. Sterilbehälter nach einem der Ansprüche 15 bis 19, **dadurch gekennzeichnet, daß** die Filtereinheit (10) in der Schließstellung am Sterilbehälter unter Vorspannung gehalten ist.

21. Sterilbehälter nach Anspruch 20, **dadurch gekennzeichnet, daß** sich ein in Längsrichtung des Lagerbolzens (90) wirkendes vorspannendes Element (92) zwischen einem der beiden Anschläge und der Lagerbuchse (28) abstützt.

22. Sterilbehälter nach einem der voranstehenden Ansprüche, **dadurch gekennzeichnet, daß** ein Dichtelement (70) zur gasdichten Lagerung der Filtereinheit (10) am Deckel (12) vorgesehen ist.

23. Sterilbehälter nach Anspruch 22, **dadurch gekennzeichnet, daß** das Dichtelement einen am Trägerelement (42, 43) gelagerten Dichtring (70) umfaßt.

24. Sterilbehälter nach einem der voranstehenden Ansprüche, **dadurch gekennzeichnet, daß** ein Überdruckventil (10) vorgesehen ist, daß das Überdruckventil so angeordnet ist, daß es in einer Grundstellung eine Schließstellung einnimmt und daß es eine Durchlaßstellung einnimmt, wenn ein Druck in einer Umgebung des Sterilbehälters einen Druck im Sterilbehälter um eine vorgegebene Druckdifferenz übersteigt.

25. Sterilbehälter nach Anspruch 24, **dadurch gekennzeichnet, daß** die Filtereinheit (10) das Überdruckventil bildet.

26. Sterilbehälter nach einem der voranstehenden Ansprüche, **dadurch gekennzeichnet, daß** ein die Filtereinheit (10) beabstandet überdeckendes Schutzelement vorgesehen ist.

27. Sterilbehälter nach Anspruch 26, **dadurch gekennzeichnet, daß** die Filtereinheit (10) auf einer Innenseite des Deckels (12) gelagert ist und daß das Schutzelement auf einer Außenseite des Deckels angeordnet ist.

28. Sterilbehälter nach einem der Ansprüche 26 oder 27, **dadurch gekennzeichnet, daß** zwischen dem Schutzelement und dem Deckel (12) mindestens eine mit der Gasaustauschöffnung in Fluidverbindung stehende Gasdurchtrittsöffnung vorgesehen ist, die so angeordnet ist, daß eine Gasströmung in einer im wesentlichen quer zur Durchlaßrichtung des Sterilfilters (52) verlaufenden Strömungsrichtung ermöglicht wird.

29. Sterilbehälter nach einem der voranstehenden Ansprüche, **dadurch gekennzeichnet, daß** eine auf der Außenseite des Deckels (12) angeordnete und von der Gasaustauschöffnung weg weisende, nach außen relativ zu einer Deckelebene abfallende Einströmkante vorgesehen ist.

30. Sterilbehälter nach einem der voranstehenden Ansprüche, **dadurch gekennzeichnet, daß** der Filterhalter (14) verdrehgesichert am Deckel (12) gelagert ist.

31. Sterilbehälter nach einem der voranstehenden Ansprüche, **dadurch gekennzeichnet, daß** das Sterilfilter ein Dauerfilter (52) ist, insbesondere ein aus Polytetrafluorethylen (PTFE) hergestelltes.

32. Sterilbehälter nach einem der voranstehenden Ansprüche, **dadurch gekennzeichnet, daß** der Deckel (12) aus einem Kunststoff hergestellt ist, insbesondere aus Polyetheretherketon (PEEK) oder Polyphenlylensulfon (PPSU).

## Claims

1. Sterile container, in particular for the holding and sterile storage of surgical instruments or material, comprising a holding space, which is formed by a container base and container walls, a lid (12) for closing the holding space and a gas exchange opening, which can be closed off by a sterile filter held in a filter holder, wherein the sterile filter (52) and the filter holder (14) form a filter unit (10), wherein the filter holder (14) is mounted on the lid (12), and wherein the sterile filter (52) is held on a carrier element (42, 43), **characterized in that** the carrier element comprises a first and a second support element (42, 43), **in that** the sterile filter (52) is held between the two support elements (42, 43), and **in that** at least one of the two support elements (42, 43) comprises supporting sections (46, 50), which span at least partially the gas exchange opening in order to support the sterile filter (52).

2. Sterile container according to claim 1, **characterized in that** the sterile filter (52) and the filter holder (14) can be releasably connected, **in that** the sterile filter (52) can be released from the filter holder (14) in a removal position and is held on the filter holder (14) in a connection position.

3. Sterile container according to one of the preceding claims, **characterized in that** the sterile filter (52), the first and the second support elements (42, 43) are non-releasably connected to one another, in particular by adhesive bonding, clamping or welding.

4. Sterile container according to claim 2 or 3, **characterized in that** there is provided a bayonet connection (38, 60) for connecting the carrier element (42, 43) and the filter holder (14) and for transferring the carrier element (42, 43) from the removal position into the connection position.

5. Sterile container according to one of claims 2 to 4, **characterized in that** there is provided a locking mechanism (40, 66) for locking the connection position of the sterile filter (52) and the filter holder (14).

6. Sterile container according to claim 5, **characterized in that** the locking mechanism comprises a latching connection (40, 66).

7. Sterile container according to one of the preceding claims, **characterized in that** the filter unit (10) comprises a cover (18) for covering the sterile filter (52) on one side.

8. Sterile container according to claim 7, **characterized in that** the cover (18) is provided with apertures (78) for allowing gas exchange through the apertures (78), and **in that** the apertures (78) are covered by aperture covers (81) in a direction which is transverse with respect to a flow-permitting direction.

9. Sterile container according to claim 7 or 8, **characterized in that** the cover (18) is spaced apart from the sterile filter (52).

10. Sterile container according to one of claims 7 to 9, **characterized in that** the cover (18) can be releasably connected to the filter holder (14), and **in that** the cover (18) can be detached from the filter holder (14) in a detachment position and is held on the filter holder (14) in a closure position.

11. Sterile container according to claim 10, **characterized in that** there is provided a second bayonet connection (32, 34, 86) for connecting the cover (18) and the filter holder (14) and for transferring the cover (18) from the detachment position into the closure position.

12. Sterile container according to claim 10 or 11, **characterized in that** there is provided a second locking mechanism (36, 37, 88) for locking the cover (18) and the filter holder (14) in the closure position.

13. Sterile container according to claim 12, **characterized in that** the second locking mechanism comprises a second latching connection (36, 37, 88).

14. Sterile container according to one of claims 7 to 13, **characterized in that** the carrier element (14) comprises a centering element (56), which can be brought into engagement with the cover (18) in order to center the cover (18) on the filter holder (14).

15. Sterile container according to one of the preceding claims, **characterized in that** the filter unit (10) is mounted movably, **in that** the filter unit (10), in a closed position, closes a flow path and, in a flow-permitting position, opens the flow path, so that gas exchange in the closed position is possible only through the sterile filter (52) and in the flow-permitting position is possible through the sterile filter (52) and/or through the flow path.

16. Sterile container according to one of the preceding claims, **characterized in that** mounting elements are provided on the filter holder (14) and on the lid (12) for mounting the filter unit (10) on the lid (12).

17. Sterile container according to claim 16, **characterized in that** the mounting elements comprise at least one mounting bolt (90) and an associated mounting bush (28), **in that** the mounting bolt (90) at each end has a stop (94) for limiting a movement of the mounting bush (28) relative to the mounting bolt (90), and **in that** the mounting bolt (90) is disposed on the lid and the mounting bush (28) is disposed on the filter holder (14), or vice versa.

18. Sterile container according to claim 17, **characterized in that** one of the two stops is formed by the lid (12) and the other stop is formed by a head (94) of the mounting bolt (90).

19. Sterile container according to one of claims 16 to 18, **characterized in that** the cover (18) covers the mounting elements.

20. Sterile container according to one of claims 15 to 19, **characterized in that** the filter unit (10) is held on the sterile container under preload in the closed position.

21. Sterile container according to claim 20, **characterized in that** an element (92) which has a preloading action in the longitudinal direction of the mounting bolt (90) is supported between one of the two stops and the mounting bush (28).

22. Sterile container according to one of the preceding claims, **characterized in that** there is provided a sealing element (70) for mounting the filter unit (10) on the lid (12) in a gastight manner.

23. Sterile container according to claim 22, **characterized in that** the sealing element comprises a sealing ring (70) mounted on the carrier element (42, 43).

24. Sterile container according to one of the preceding claims, **characterized in that** there is provided a pressure-relief valve (10), **in that** the pressure-relief valve is disposed in such a way that in a basic position it adopts a closed position, and **in that** it adopts a flow-permitting position when a pressure in the vicinity of the sterile container exceeds a pressure in the sterile container by a predetermined pressure difference.

25. Sterile container according to claim 24, **characterized in that** the filter unit (10) forms the pressure-relief valve.

26. Sterile container according to one of the preceding claims, **characterized in that** there is provided a protective element which covers the filter unit (10) at a spacing therefrom.

27. Sterile container according to claim 26, **characterized in that** the filter unit (10) is mounted on an inner side of the lid (12), and **in that** the protective element is disposed on an outer side of the lid.

28. Sterile container according to claim 26 or 27, **characterized in that** between the protective element and the lid (12) there is provided at least one opening for the passage of gas, which is in fluid communication with the gas exchange opening and is disposed in such a way that gas flow is possible in a direction of flow running substantially transversely with respect to the flow-permitting direction of the sterile filter (52).

29. Sterile container according to one of the preceding claims, **characterized in that** there is provided an inflow edge which is disposed on the outer side of the lid (12), faces away from the gas exchange opening and slopes downward toward the outside relative to a lid plane.

30. Sterile container according to one of the preceding claims, **characterized in that** the filter holder (14) is mounted on the lid (12) in a manner which is secured against rotation.

31. Sterile container according to one of the preceding claims, **characterized in that** the sterile filter is a long-term filter (52), in particular made from polytetrafluoroethylene (PTFE).

32. Sterile container according to one of the preceding claims, **characterized in that** the lid (12) is made from a plastic, in particular from polyether ether ketone (PEEK) or polyphenylene sulfone (PPSU).

## Revendications

1. Récipient ou réceptacle stérile, notamment destiné à recevoir et à conserver de manière stérile des instruments, ustensiles ou matériaux chirurgicaux, comprenant un logement de réception formé par un fond de récipient et des parois de récipient, un couvercle (12) pour fermer le logement de réception, et une ouverture d'échange de gaz, qui peut être fermée par un filtre stérile maintenu dans un support de filtre, le filtre stérile (52) et le support de filtre (14) formant une unité de filtre (10), et le support de filtre (14) étant monté sur le couvercle (12), et le filtre stérile (52) étant maintenu par un élément porteur (42, 43),
**caractérisé en ce que** l'élément porteur comprend un premier et un deuxième élément de soutien (42, 43), le filtre stérile (52) étant maintenu entre les deux éléments de soutien (42, 43), et au moins l'un des deux éléments de soutien (42, 43) comprenant des tronçons d'appui (46, 50) pour le soutien du filtre stérile (52), qui surmontent au moins partiellement l'ouverture d'échange de gaz.

2. Récipient stérile selon la revendication 1, **caractérisé en ce que** le filtre stérile (52) et le support de filtre (14) peuvent être reliés de manière amovible, et **en ce que** le filtre stérile (52) peut, dans une position de retrait ou d'enlèvement, être détaché du support de filtre (14), et dans une position de liaison, être maintenu sur le support de filtre (14).

3. Récipient stérile selon l'une des revendications précédentes, **caractérisé en ce que** le filtre stérile (52), le premier et le deuxième élément de soutien (42, 43) sont liés mutuellement de manière inséparable, notamment par collage, serrage ou soudage.

4. Récipient stérile selon l'une des revendications 2 ou 3, **caractérisé en ce qu'**il est prévu un système de liaison à baïonnette (38, 60) pour relier l'élément porteur (42, 43) et le support de filtre (14) et pour transférer l'élément porteur (42, 43) de la position de retrait ou d'enlèvement à la position de liaison.

5. Récipient stérile selon l'une des revendications 2 à 4, **caractérisé en ce qu'**il est prévu un mécanisme de verrouillage (40, 66) pour verrouiller la position de liaison du filtre stérile (52) et du support de filtre (14).

6. Récipient stérile selon la revendication 5, **caractérisé en ce que** le mécanisme de verrouillage comprend une liaison par encliquetage (40, 66).

7. Récipient stérile selon l'une des revendications précédentes, **caractérisé en ce que** l'unité de filtre (10) comprend un élément de recouvrement (18) pour recouvrir le filtre stérile (52) d'un côté.

8. Récipient stérile selon la revendication 7, **caractérisé en ce que** l'élément de recouvrement (18) est pourvu d'ouvertures de passage (78) en vue de permettre un échange de gaz à travers les ouvertures de passage (78), et **en ce que** les ouvertures de passage (78) sont, dans une direction transversale à une direction de passage, recouvertes d'éléments de recouvrement d'ouverture de passage (81).

9. Récipient stérile selon l'une des revendications 7 ou 8, **caractérisé en ce que** l'élément de recouvrement (18) est espacé du filtre stérile (52).

10. Récipient stérile selon l'une des revendications 7 à 9, **caractérisé en ce que** l'élément de recouvrement (18) peut être relié de manière amovible avec le support de filtre (14), et **en ce que** l'élément de recouvrement (18) peut être retiré du support de filtre (14), dans une position d'enlèvement, et est maintenu sur le support de filtre (14), dans une position de fermeture.

11. Récipient stérile selon la revendication 10, **caractérisé en ce qu'**il est prévu une deuxième liaison à baïonnette (32, 34, 86) pour relier l'élément de recouvrement (18) et le support de filtre (14), et pour transférer l'élément de recouvrement (18) de la position d'enlèvement dans la position de fermeture.

12. Récipient stérile selon l'une des revendications 10 ou 11, **caractérisé en ce qu'**il est prévu un deuxième mécanisme de verrouillage (36, 37, 88) pour verrouiller l'élément de recouvrement (18) et le support de filtre (14) dans la position de fermeture.

13. Récipient stérile selon la revendication 12, **caractérisé en ce que** le deuxième mécanisme de verrouillage comprend une deuxième liaison par encliquetage (36, 37, 88).

14. Récipient stérile selon l'une des revendications 7 à 13, **caractérisé en ce que** l'élément porteur (14) comprend un élément de centrage (56) qui peut être amené en prise avec l'élément de recouvrement (18), et est destiné au centrage de l'élément de recouvrement (18) sur le support de filtre (14).

15. Récipient stérile selon l'une des revendications précédentes, **caractérisé en ce que** l'unité de filtre (10) est montée de manière mobile, et **en ce que** l'unité de filtre (10), dans une position de fermeture, ferme un chemin d'écoulement, et dans une position de passage, ouvre le chemin d'écoulement, de sorte qu'un échange de gaz n'est possible, dans la position de fermeture, qu'à travers le filtre stérile (52), et dans la position de passage, à travers le filtre stérile (52) et/ou par le chemin d'écoulement.

16. Récipient stérile selon l'une des revendications précédentes, **caractérisé en ce que** des éléments de palier de montage sont prévus sur le support de filtre (14) et sur le couvercle (12), pour le montage de l'unité de filtre (10) sur le couvercle (12).

17. Récipient stérile selon la revendication 16, **caractérisé en ce que** les éléments de palier de montage comprennent au moins un tenon de palier (90) et une douille de palier (28) associée, **en ce que** le tenon de palier (90) présente respectivement aux extrémités, une butée (94) pour limiter un mouvement de la douille de palier (28) par rapport au tenon de palier (90), et **en ce que** le tenon de palier (90) est agencé sur le couvercle et la douille de palier (28) est agencée sur le support de filtre (14), ou inversement.

18. Récipient stérile selon la revendication 17, **caractérisé en ce que** l'une des deux butées est formée par le couvercle (12) et l'autre butée est formée par une tête (94) du tenon de palier (90).

19. Récipient stérile selon l'une des revendications 16 à 18, **caractérisé en ce que** l'élément de recouvrement (18) recouvre les éléments de palier de montage.

20. Récipient stérile selon l'une des revendications 15 à 19, **caractérisé en ce que** l'unité de filtre (10), dans la position de fermeture, est maintenue sous précontrainte sur le récipient stérile.

21. Récipient stérile selon la revendication 20, **caractérisé en ce qu'**un élément (92) de précontrainte agissant dans la direction longitudinale du tenon de palier (90) s'appuie entre l'une des deux butées et la douille de palier (28).

22. Récipient stérile selon l'une des revendications précédentes, **caractérisé en ce qu'**il est prévu un élément d'étanchéité (70) pour le montage étanche aux gaz de l'unité de filtre (10) sur le couvercle (12).

23. Récipient stérile selon la revendication 22, **caractérisé en ce que** l'élément d'étanchéité comprend une bague d'étanchéité (70) montée dans l'élément porteur (42, 43).

24. Récipient stérile selon l'une des revendications précédentes, **caractérisé en ce qu'**il est prévu une soupape de surpression (10), **en ce que** la soupape de surpression est agencée de façon à prendre une position de fermeture dans la position de base, et **en ce qu'**elle prend une position de passage lorsqu'une pression dans un environnement du récipient stérile dépasse une pression dans le récipient stérile d'une valeur de différence de pression prescrite.

25. Récipient stérile selon la revendication 24, **caractérisé en ce que** l'unité de filtre (10) forme la soupape de surpression.

26. Récipient stérile selon l'une des revendications précédentes, **caractérisé en ce qu'**il est prévu un élément de protection recouvrant à distance l'unité de filtre (10).

27. Récipient stérile selon la revendication 26, **caractérisé en ce que** l'unité de filtre (10) est montée sur un côté intérieur du couvercle (12), et **en ce que** l'élément de protection est agencé sur un côté extérieur du couvercle.

28. Récipient stérile selon l'une des revendications 26 ou 27, **caractérisé en ce qu'**il est prévu entre l'élément de protection et le couvercle (12), au moins une ouverture de passage de gaz, qui est en liaison d'écoulement de fluide avec l'ouverture d'échange de gaz, et est agencée de manière à permettre un écoulement de gaz dans une direction d'écoulement s'étendant sensiblement transversalement à la direction de passage du filtre stérile (52).

29. Récipient stérile selon l'une des revendications précédentes, **caractérisé en ce qu'**il est prévu une rampe d'entrée d'écoulement agencée sur le côté extérieur du couvercle (12) et s'éloignant de l'ouverture d'échange de gaz, et s'abaissant vers l'extérieur par rapport à un plan du couvercle.

30. Récipient stérile selon l'une des revendications précédentes, **caractérisé en ce que** le support de filtre (14) est monté de manière arrêtée en rotation sur le couvercle (12).

31. Récipient stérile selon l'une des revendications précédentes, **caractérisé en ce que** le filtre stérile est un filtre permanent (52), notamment fabriqué en polytétrafluoroéthylène (PTFE).

32. Récipient stérile selon l'une des revendications précédentes, **caractérisé en ce que** le couvercle (12) est fabriqué en une matière plastique, en particulier en polyétheréthercétone (PEEK) ou en polyphénylsulfone (PPSU).
